# EUROPEAN PATENT APPLICATION

(11) **EP 4 566 634 A1**
(43) Date of publication of application: **11.06.2025**
(21) Application number: 23852795.6
(22) Date of filing: 24.07.2023
(51) Int. Cl.: A61L 2/10, A61L 2/26

(54) **STERILIZATION MODULE**

(30) Priority: 09.08.2022 KR 20220099338
(71) Applicant: LG Electronics Inc., Yeongdeungpo-gu Seoul 07336 (KR)
(72) Inventor: HONG, Seongkyeol, Seoul 08592 (KR); MOON, Sunyoung, Seoul 08592 (KR); LEE, Sangheon, Seoul 08592 (KR); KIM, Seonyeob, Daegu 42612 (KR); YUN, Sangwon, Daegu 41427 (KR); RYU, Jeungsoo, Daegu 42763 (KR); HA, Changwon, Daegu 41408 (KR); KIM, Kyungkwan, Daegu 42713 (KR)
(74) Representative: Vossius & Partner Patentanwälte Rechtsanwälte mbB
(86) International application number: PCT/KR2023/010650
(87) International publication number: WO 2024/034914

(57) **Abstract**

An embodiment of the present disclosure relates to a sterilization module comprising: a light source module that generates light; a light guide which extends in a first direction and into which the light is incident; and a body having an upper surface that is open and supporting the light guide disposed therein, wherein the light guide includes a first side and a second side disposed opposite the first side in the first direction, and the light source module is disposed on the first side of the light guide to allow the light to be incident on the light guide.

## Description

### TECHNICAL FIELD

The present disclosure relates to a sterilization module.

### BACKGROUND ART

Air conditioners are apparatuses that adjust an indoor air temperature to a temperature set by a user to cool and heat the indoor air based on the principle of refrigeration cycle. The air conditioners may be classified into wall-mounted types, stand-alone types, and duct types depending on an installation method of an indoor unit.

Each air conditioner may be equipped with a filter to filter out foreign substances such as fine dust and particulate matter floating in the air to be inhaled. As an air conditioner is used repeatedly, foreign substances accumulate in the filter, which causes problems such as reduced air purifying performance, unpleasant odors, and decreased airflow.

Accordingly, a sterilizing device for sterilizing the filter may be installed inside the air conditioner.

### DETAILED DESCRIPTION OF INVENTION

### TECHNICAL PROBLEMS

An object to be solved by the present disclosure is to provide a sterilization module capable of uniformly sterilizing a surface of a filter vulnerable to contamination, and an air conditioner including the same.

Another object of the present disclosure is to provide a sterilization module capable of radiating light having uniform illuminance, and an air conditioner including the same.

Another object of the present disclosure is to provide a sterilization module capable of adjusting a radiation angle in a longitudinal direction of the sterilization module, and an air conditioner including the same.

Another object of the present disclosure is to provide a sterilization module capable of adjusting a radiation angle in a circumferential direction of the sterilization module, and an air conditioner including the same.

Another object of the present disclosure is to provide a sterilization module capable of minimizing light loss, and an air conditioner including the same.

Another object of the present disclosure is to provide a sterilization module capable of irradiating light to an inner side of a bent portion of a bent filter, and an air conditioner including the same.

Another object of the present disclosure also is to provide a sterilization module capable of sterilizing an entire area using a single light source when it comes to sterilizing a long surface or a wide surface, and an air conditioner including the same.

Objects of the present disclosure are not limited to the objects mentioned above, and other objects not mentioned will be clearly understood by those skilled in the art from the description below.

### TECHNICAL SOLUTION

In order to achieve the aforementioned objects, a sterilization module according to embodiments of the present disclosure may include: a light source module generating light; a light guide extending in a first direction and into which light is incident; and a body having an open upper side and supporting the light guide disposed therein. The light guide may include a first side and a second side disposed opposite the first side in the first direction, and the light source module may be disposed at the first side of the light guide to allow light to be incident on the light guide. Accordingly, it is possible to reduce a material cost of the sterilization module and simplify the structure, such as wiring, by using a single light source.

The light guide may have a cross-section formed in a circular shape. Accordingly, it is possible to implement a sterilization module that radiates a linear light source.

The light source module may include a light source that generates light having a wavelength of 100 nm to 280 nm. The light guide may be formed of a material having a high light transmittance. Accordingly, it is possible to improve sterilizing power.

The light guide may include: a first curved section connecting the first side and the second side and formed as a curved surface; and a second curved section connecting the first side and the second side, formed as a curved surface, and positioned below the first curved section. A reflection pattern may be formed in the second curved section. The light incident into the light guide may be reflected from the second curved section and emitted to the first curved section. Accordingly, it is possible to enable total reflection in a longitudinal direction of the light guide.

The reflection pattern may include a plurality of first reflection patterns extending in the first direction and spaced apart from each other in the first direction. A separation distance between the plurality of first reflection patterns may decrease in a direction away from the light source module. Accordingly, it is possible to irradiate light with uniform illuminance by controlling a reflection pattern.

The reflection pattern may include a plurality of second reflection patterns extending in a second direction, which is perpendicular to the first direction and spaced apart from each other in the first direction. A separation distance between the plurality of second reflection patterns may decrease in a direction away from the light source module. Accordingly, it is possible to irradiate light with uniform illuminance by controlling a reflection pattern.

The body may include a grip member coupled to a bottom plate of the body and gripping the light guide. Accordingly, it is possible to fix the light guide to the body.

The body may include a bottom plate, a first side plate extending upward from the bottom plate and having the first direction as a longitudinal direction, and a second side plate extending upward from the bottom plate and disposed opposite the first side plate. A reflector may be disposed at the bottom plate, the first side plate, and the second side plate. Accordingly, by placing the reflector on the body, it is possible to reduce light loss in a direction toward the reflection pattern of the light guide.

The reflector may contain aluminum. By using a reflector formed of a material having a high reflectivity for UV-C wavelength, it is possible to reduce light loss in a direction toward the reflection pattern of the light guide.

The reflector may include a first reflector disposed at the first side plate and a second reflector disposed at the second side plate. A distance between the first reflector and the second reflector in a second direction perpendicular to the first direction may increase upward along a third direction perpendicular to both the first and second directions. Accordingly, it is possible to adjust a radiation angle by adjusting the reflector disposed on the body instead of the light guide.

At least a portion of the reflector may be formed as a curved surface. Accordingly, it is possible to adjust a radiation angle by adjusting the reflector disposed on the body instead of the light guide.

The light guide may include one end portion including the first side and the other end portion including the second side. The sterilization module may further include a fixing member for fixing the other end portion of the light guide. The fixing member may be formed with a groove into which the other end portion of the light guide is inserted. Accordingly, it is possible to prevent a cylindrical light guide from rotating.

The body may include a bottom plate, a first side plate extending upward from the bottom plate, a second side plate disposed opposite the first side plate, a third side plate connecting the first side plate and the second side plate and facing the first side of the light guide, and a fourth side plate disposed opposite the third side plate. The other end portion of the light guide may include a first recessed portion recessed inward from at least a portion of an outer peripheral surface of the other end portion of the light guide and facing the first side plate of the body. The other end portion of the light guide may include a second recessed portion recessed inward from at least a portion of the outer peripheral surface of the other end portion of the light guide and facing the second side plate of the body. The first recessed portion and the second recessed portion may be formed as flat surfaces. Accordingly, it is possible to prevent a cylindrical light guide from rotating.

The other end portion of the light guide may include a recessed portion recessed from at least a portion of an outer peripheral surface of the other end portion of the light guide and faces the bottom plate of the body. The recessed portion may be formed as a flat surface. Accordingly, it is possible to prevent a cylindrical light guide from rotating.

The light source module may include a substrate; and a light source mounted on the substrate. The body may include a first side plate facing a first side of a light guide, and the first side plate of the body may include a first surface facing the first side of the light guide, a second surface disposed opposite the first surface, and a hole penetrating the first surface and the second surface. The substrate may be disposed at the second surface of the first side plate of the body, and the light source may be disposed at the hole of the first side plate. Accordingly, it is possible to separate spaces where the light source module and the light guide are installed, and by separating the spaces to protect the substrate and utilizing a hole, it is possible to minimize a short circuit between the light source mounted on the substrate and the substrate.

The light source module may include a connector. The substrate may include a first portion disposed at the first side plate of the body, and a second portion bent from the first portion and at which the connector is disposed. The sterilization module may further include a cover coupled to the body and covering the first portion and the second portion of the substrate. Accordingly, it is possible to project the substrate, the substrate mounting components such as the light source and connector.

Specific details of other embodiments are included in the detailed description and drawings.

### EFFECT OF INVENTION

According to a sterilization module of the present disclosure, one or more of the following effects are achieved.

First, it is possible to uniformly sterilize a surface of a filter vulnerable to contamination.

Second, it is possible to radiate light with uniform illuminance.

Third, it is possible to adjust a radiation angle in a longitudinal direction of the sterilization module.

Fourth, it is possible to adjust a radiation angle in a circumferential direction of the sterilization module.

Fifth, it is possible to minimize light loss.

Sixth, it is possible to irradiate light to an inner side of a bent portion of a bent filter.

Seventh, when it comes to sterilizing a long surface or a wide surface, it is possible to sterilize an entire area using a single light source.

Eighth, by using a single light source, it is possible to reduce a material cost of the module and simplify a wiring structure.

Effects of the present disclosure are not limited to the effects mentioned above, and other effects not mentioned will be clearly understood by those skilled in the art from the description of the claims.

### BRIEF DESCRIPTION OF THE DRAWING

FIG. 1 is a perspective view of a sterilization module according to one embodiment of the present disclosure.
FIG. 2 is an enlarged perspective view of a portion of a sterilization module according to one embodiment of the present disclosure.
FIG. 3 is a perspective view of a portion of the sterilization module of FIG. 2 viewed from a different direction.
FIG. 4 is a view in which a cover is removed from FIG. 2.
FIG. 5 is a view in which the cover and a light guide are removed from FIG. 3.
FIGS. 6 and 7 are cross-sectional views of a sterilization module according to one embodiment of the present disclosure.
FIGS. 8 and 9 are schematic views illustrating a reflection pattern applied to a light guide of a sterilization module according to one embodiment of the present disclosure.
FIG. 10 is a view illustrating an optical path in a sterilization module according to one embodiment of the present disclosure.
FIGS. 11 to 13 are views showing various examples of a reflection pattern of a sterilization module according to one embodiment of the present disclosure.
FIG. 14 and FIG. 15 are views showing an rotation prevention structure of a light guide of a sterilization module according to one embodiment of the present disclosure.
FIGS. 16 and 17 are views showing other examples of a rotation prevention structure of a light guide of a sterilization module according to one embodiment of the present disclosure.
FIG. 18 is a view showing an air conditioner according to one embodiment of the present disclosure.
FIG. 19 is a view showing an example of a sterilization module of FIG. 1 being arranged in an air conditioner according to one embodiment of the present disclosure.
FIGS. 20 and 21 are views showing a radiation angle of the sterilization module in FIG. 19.
FIG. 22 is a view showing another example in which the sterilization module of FIG. 1 is arranged in an air conditioner according to one embodiment of the present disclosure.
FIG. 23 is a view showing the radiation angle of the sterilization module in FIG. 22.

### MODE FOR CARRYING OUT THE INVENTION

Hereinafter, embodiments disclosed in this specification will be described in detail with reference to the accompanying drawings. The same or equivalent elements may be provided with the same or similar reference numbers, and description thereof will not be repeated.

In the following description, a suffix such as "module" and "unit" may be used to refer to elements or components, and the use of such a suffix herein is merely intended to facilitate description of the specification, and the suffix itself is not intended to give any special meaning or function.

In addition, in the following description of the embodiments, a detailed description of known functions and configurations incorporated herein will be omitted when it may impede the understanding of the embodiments. In addition, the accompanying drawings are used to help easily understand various technical features and it should be understood that the embodiments presented herein are not limited by the accompanying drawings, and the present disclosure should be construed to extend to any alterations, equivalents and substitutes in addition to those which are particularly set out in the accompanying drawings.

While terms including ordinal numbers, such as "first" and "second," etc., may be used to describe various elements, such elements are not limited by the above terms. These terms are generally only used to distinguish one element from another.

It will be understood that when an element is referred to as being "connected with" another element, the element may be connected with the other element or intervening elements may also be present. In contrast, when an element is referred to as being "directly connected with" another element, there are no intervening elements present. When an element is referred to as being "directly connected" or "directly accessed" to other component, it should be understood that there is no element therebetween.

Unless stated otherwise, an expression of singularity is intended to include expressions of plurality.

Hereinafter, a sterilization module 10 according to one embodiment of the present disclosure will be described in detail with reference to the drawings.

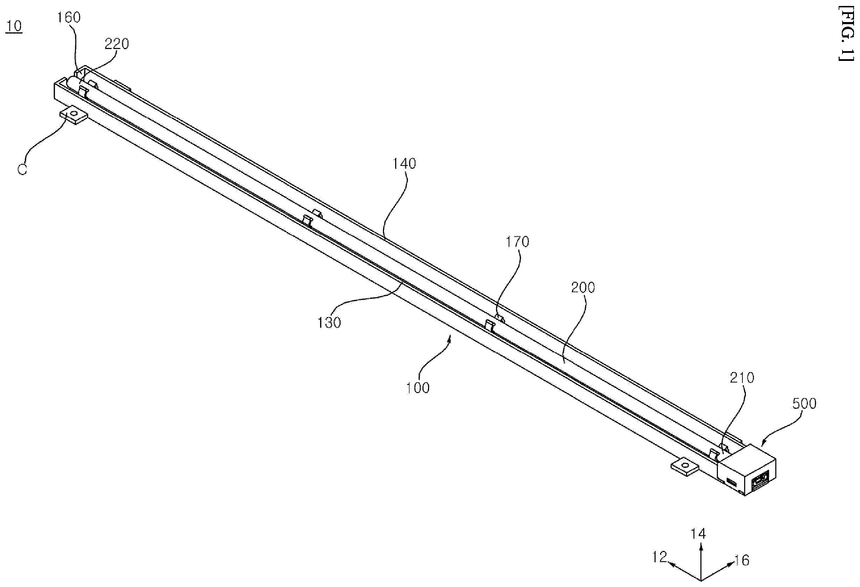

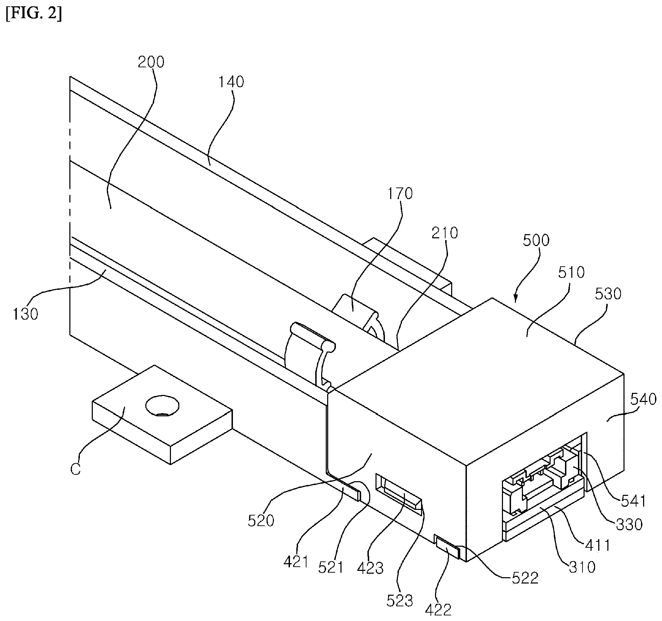

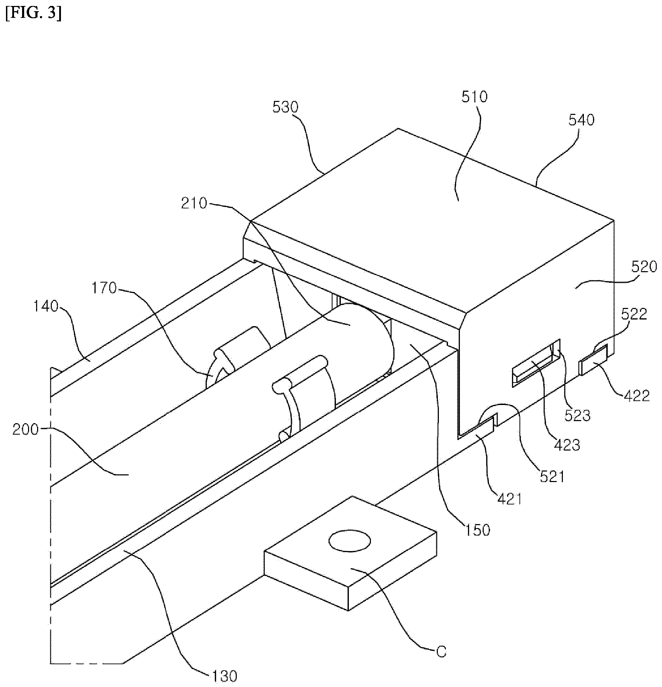

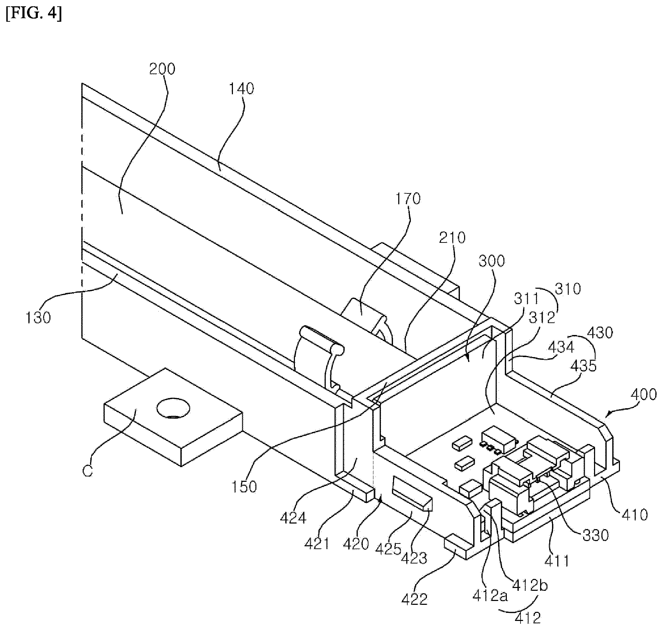

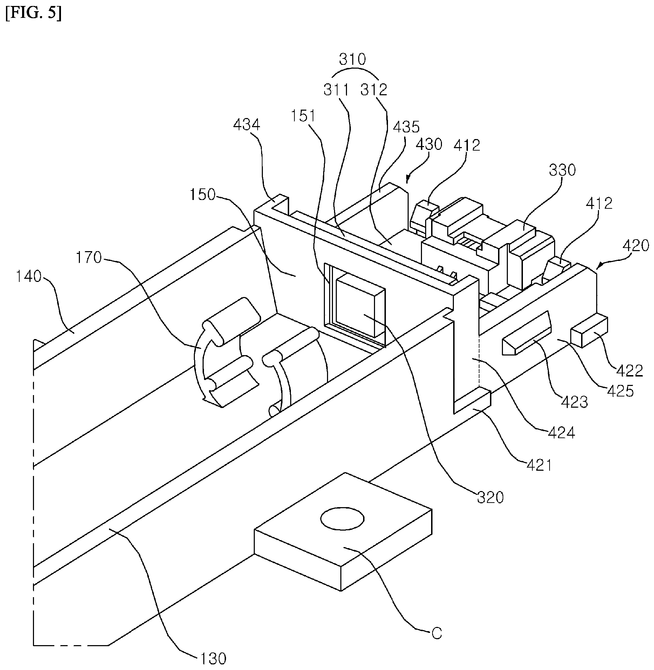

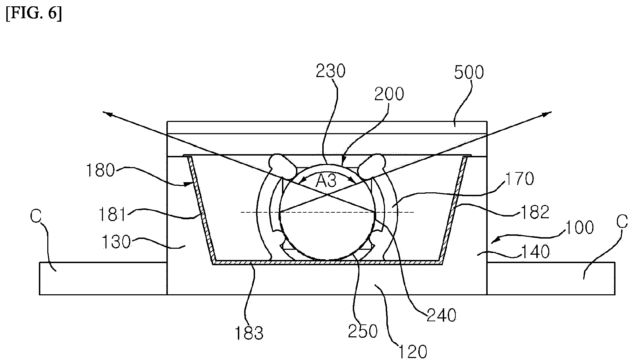

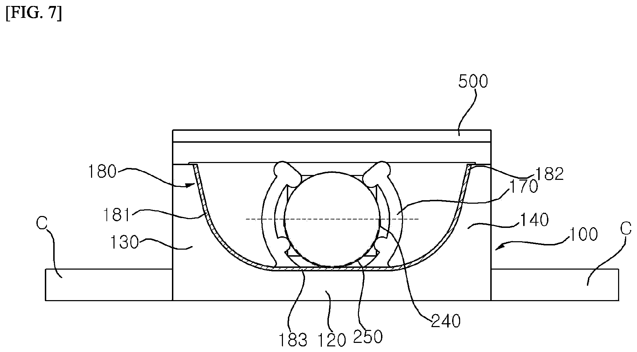

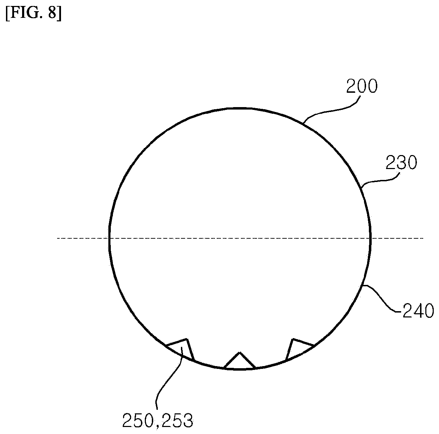

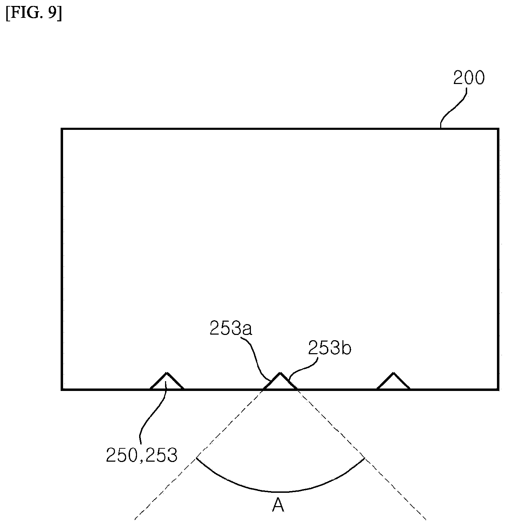

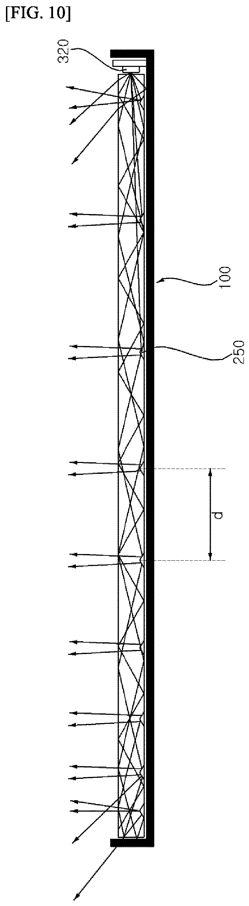

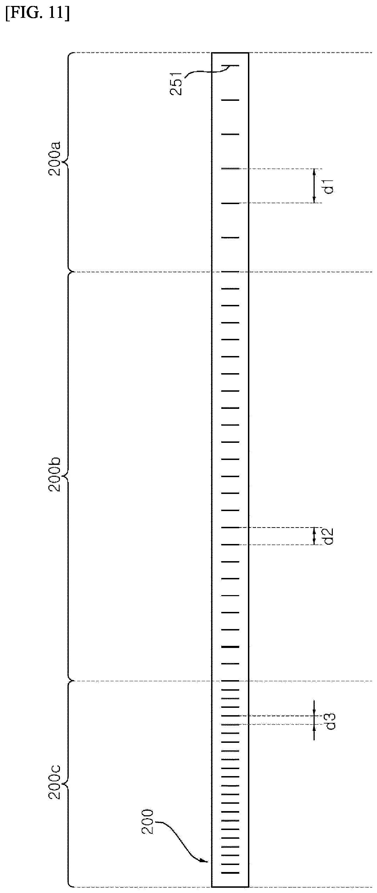

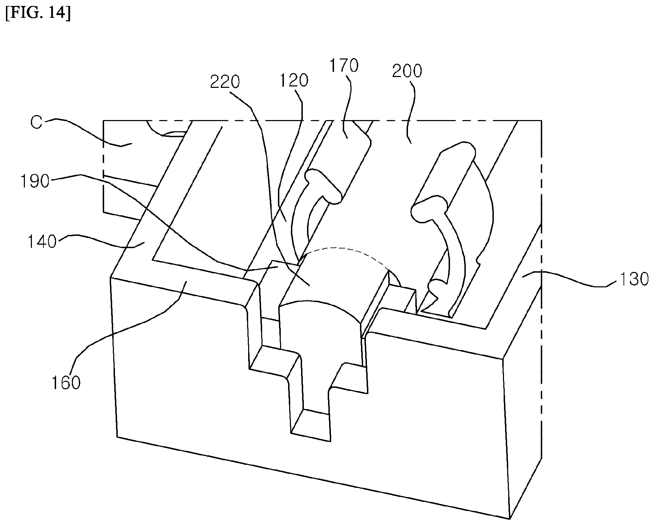

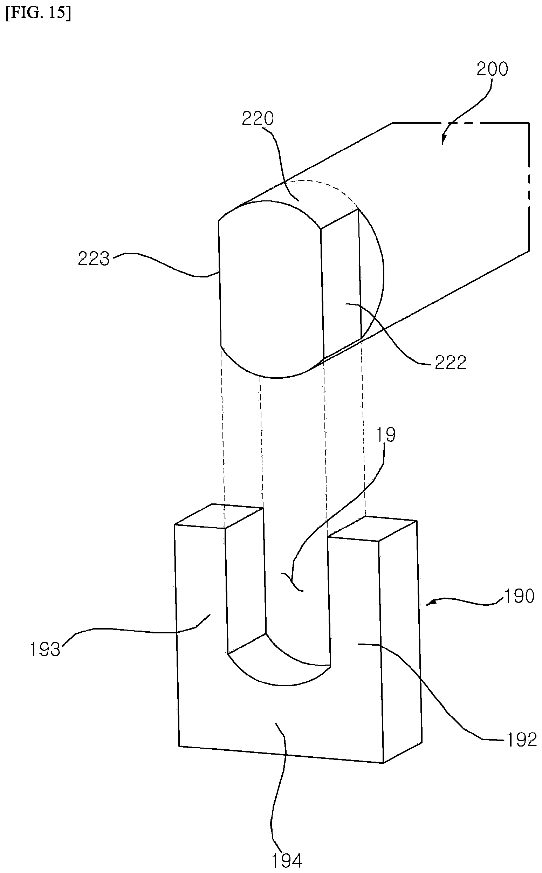

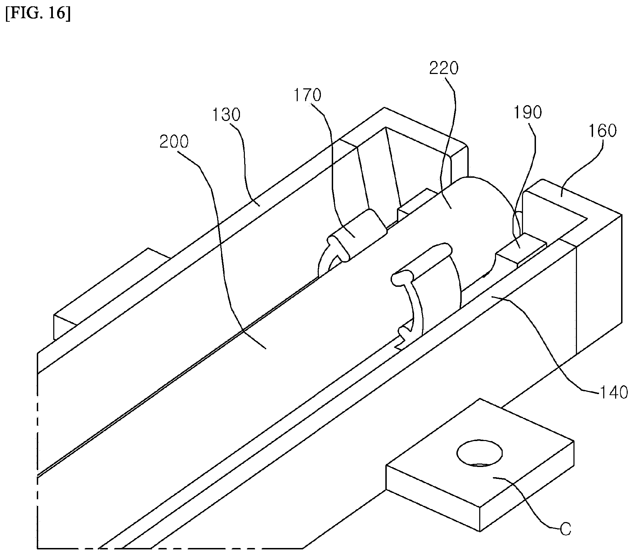

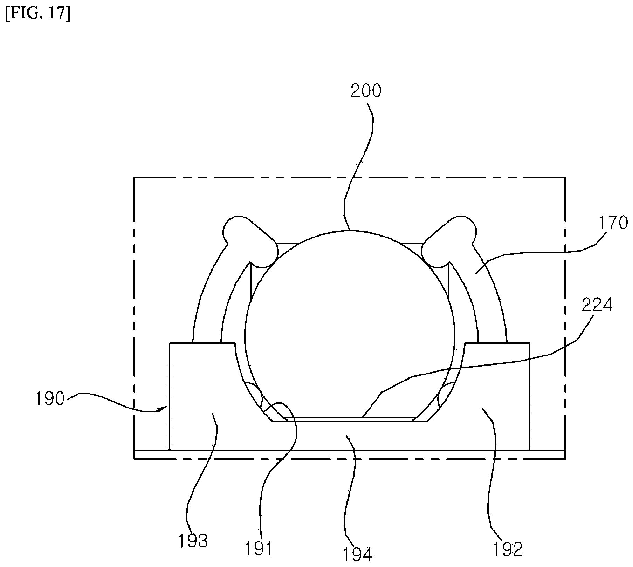

FIG. 1 is a perspective view of a sterilization module according to one embodiment of the present disclosure. FIG. 2 is an enlarged perspective view of a portion of a sterilization module according to one embodiment of the present disclosure. FIG. 3 is a perspective view of a portion of the sterilization module of FIG. 2 viewed from a different direction. FIG. 4 is a view in which a cover is removed from FIG. 2. FIG. 5 is a view in which the cover and a light guide are removed from FIG. 3. FIGS. 6 and 7 are cross-sectional views of a sterilization module according to one embodiment of the present disclosure. FIGS. 8 and 9 are schematic views illustrating a reflection pattern applied to a light guide of a sterilization module according to one embodiment of the present disclosure. FIG. 10 is a view illustrating an optical path in a sterilization module according to one embodiment of the present disclosure. FIGS. 11 to 13 are views showing various examples of a reflection pattern of a sterilization module according to one embodiment of the present disclosure. FIG. 14 and FIG. 15 are views showing an rotation prevention structure of a light guide of a sterilization module according to one embodiment of the present disclosure. FIGS. 16 and 17 are views showing other examples of a rotation prevention structure of a light guide of a sterilization module according to one embodiment of the present disclosure.

Referring to FIGS. 1 to 5, the sterilization module 10 may include a body 100. The body 100 may form the exterior of the sterilization module 10. The body 100 may have a quadrangular cross-section. For example, the body 100 may be formed in a rectangular shape having a first direction 12 as a longitudinal direction. The body 100 may extend in the same direction as a light guide 200 to be described. Hereinafter, the longitudinal direction of the body 100 or the longitudinal direction of the light guide 200 may be referred to as the first direction 12, a direction perpendicular to the first direction 12 may be referred to as a second direction 14, and a direction perpendicular to both the first direction 12 and the second direction 14 may be referred to as a third direction 16.

The body 100 may have a space formed inside to accommodate the light guide 200. The body 100 may support the light guide 200. The body 100 may fix the light guide 200. The body 100 may have an open upper side. The open upper side of the body 100 may be a passage which light emitted from the light guide 200 passes through. The open upper side of the body 100 may be opened in a direction toward an object to be sterilized. The body 100 may serve to help a light emitted from the light guide 200 to be directed toward the upper side of the body 100. Related details thereof will be described later.

The body 100 may include a bottom plate 120 and a plurality of side plates extending upward from the bottom plate 120. The plurality of side plates may include a first side plate 130, a second side plate 140 disposed opposite the first side plate 130, a third side plate 150 connecting the first side plate 130 and the second side plate 140, and a fourth side plate 160 disposed opposite the third side plate 150. The first side plate 130 and the second side plate 140 may each have a length corresponding to a length of the body 100 in the first direction 12. The third side plate 150 and the fourth side plate 160 may be formed with a shorter length than that of the first side plate 130. The third side plate 150 and the fourth side plate 160 may each have a corresponding length in the third direction 16. The opposite side of the bottom plate 120 may be the open upper side of the body 100.

Referring to FIG. 5, a hole 151 may be formed in the third side plate 150 of the body 100. The third side plate 150 of the body 100 may include a first surface facing the light guide 200 and a second surface disposed opposite the first surface. The hole 151 may penetrate the first and second surfaces of the third side plate 150 of the body 100. A light source 320 of a light source module 300 described later may be disposed in the hole 151. The hole 151 may be formed in a shape corresponding to that of the light source 320. For example, the light source 320 may have a quadrangular shape, and the hole 151 may be formed in a quadrangular shape. The hole 151 may have a size corresponding to that of the light source 320. Alternatively, the hole 151 may have a larger size than that of the light source 320. The hole 151 may be formed at a portion of a light incident surface 211 of the light guide 200 that faces the third side plate 150 of the body 100 when the light guide 200 is disposed at the body 100. Accordingly, the light source 320 disposed in the hole 151 may enter the interior of the light guide 200 through the light incident surface 211 of the light guide 200.

Referring to FIGS. 1 to 5, a grip member 170 for fixing the light guide 200 may be disposed in the body 100. The grip member 170 may hold the light guide 200. The grip member 170 may grip at least a portion of the light guide 200. The grip member 170 may be coupled to the bottom plate 120. The grip member 170 may include a first gripping portion 171 and a second gripping portion 174. The first gripping portion 171 may be spaced apart from the second gripping portion 174. The first gripping portion 171 may be disposed closer to the first side plate 130 than the second gripping portion 174. The second gripping portion 174 may be disposed closer to the second side plate 140 than the first gripping portion 171. The light guide 200 may be disposed between the first gripping portion 171 and the second gripping portion 174.

The first gripping portion 171 and the second gripping portion 174 may have the same shape. The first gripping portion 171 and the second gripping portion 174 may be symmetrical to each other with respect to an axis extending in the first direction 12. The first gripping portion 171 and the second gripping portion 174 may be symmetrical to each other with respect to an imaginary flat surface formed by an axis extending in the first direction 12 and an axis extending in the second direction 14.

The first gripping portion 171 may include a body portion 172 and a projection 173 protruding from at least a portion of the body portion 172. The body portion 172 may surround at least a portion of the light guide 200. The body portion 172 may be spaced apart from the light guide 200. The body portion 172 may include a round plate having a curved shape. The body portion 172 may have a curvature corresponding to a curvature of the light guide 200. However, aspects of the present disclosure are not limited to this. The projection 173 may protrude from an inner surface of the body portion 172. The projection 173 may come into contact with the light guide 200. The projection 173 may include a first projection 1731 and a second projection 1732. The first projection 1731 and the second projection 1732 may be spaced apart along an extension direction of the body portion 172. The first projection 1731 may be formed at an upper end of the body portion 172. The first projection 1731 may be positioned higher than the second projection 1732. The first projection 1731 may come into contact with a first curved section 230 of the light guide 200 to be described later. The first projection 1731 may protrude at an incline. The first projection 1731 may protrude at an incline in a direction toward the light guide 200. The second projection 1732 may be formed below the first projection 1731. The second projection 1732 may be spaced apart from the bottom plate 120. The second projection 1732 may come into contact with a second curved section 240 of the light guide 200 to be described later. The second projection 1732 may protrude at an incline. The second projection 1732 may protrude at an incline in a direction toward the light guide 200. The second projection 1732 may protrude at an incline in a direction different from an inclined direction of the first projection 1731. The second projection 1732 may protrude at an incline in a direction intersecting with the inclined direction of the first projection 1731.

The second gripping portion 174 may include a body portion 175 and a projection 176 protruding from at least a portion of the body portion 175. The body portion 175 may surround at least a portion of the light guide 200. The body portion 175 may be spaced apart from the light guide 200. The body portion 175 may include a round plate having a curved shape. The body portion 175 may have a curvature corresponding to the curvature of the light guide 200. However, aspects of the present disclosure are not limited to this. The projection 176 may protrude from an inner surface of the body portion 175. The projection 176 may come into contact with the light guide 200. The projection 176 may include a third projection 1761 and a fourth projection 1762. The third projection 1761 and the fourth projection 1762 may be spaced apart along an extension direction of the body portion 175. The third projection 1761 may be formed at an upper end of the body portion 175. The third projection 1761 may be positioned higher than the second projection 1762. The third projection 1761 may come into contact with the first curved section 230 of the light guide 200. The third projection 1761 may protrude at an incline. The third projection 1761 may protrude at an incline in a direction toward the light guide 200. Referring to FIG. 6 or FIG. 7, the third projection 1761 may have an inclined direction corresponding to the inclined direction of the second projection 1732. The third projection 1761 may be point-symmetrical with the second projection 1732. The third projection 1761 may overlap the first projection 1731 in the third direction 16. The fourth projection 1762 may be formed below the third projection 1761. The fourth projection 1762 may be spaced apart from the bottom plate 120. The fourth projection 1762 may come into contact with the second curved section 240 of the light guide 200 to be described later. The fourth projection 1762 may protrude at an incline. The fourth projection 1762 may protrude at an incline in a direction toward the light guide 200. The fourth projection 1762 may protrude at an incline in a direction different from the inclined direction of the third projection 1761. The fourth projection 1762 may protrude at an incline in a direction intersecting with the inclined direction of the third projection 1761. Referring to FIG. 6 or FIG. 7, the fourth projection 1762 may have an inclined direction corresponding to the inclined direction of the first projection 1731. The fourth projection 1762 may be point-symmetrical with the first projection 1731. The fourth projection 1762 may overlap the second projection 1732 in the second direction 16.

Through the structure of the grip member 170 described above, even if the sterilization module 10 is installed at various angles, the light guide 200 may be firmly fixed to the body 100, and the light guide 200 may be prevented from being removed from the body 100.

The grip member 170 may include a plurality of grip members 170. The plurality of picture members 170 may be spaced apart from each other in the first direction. Accordingly, even if a length of the light guide 200 is increased according to a length of a required light source, the light guide 200 may be firmly fixed to the body 100, and the light guide 200 may be prevented from being removed from the body 200.

A coupling part C may be coupled to the body 100. The coupling part C may be disposed at the first side plate 120 and the second side plate 140 of the body 100. The sterilization module 10 may be coupled at an installation location due to the coupling part C. A hole for screwing may be formed in the coupling part C. The coupling part C may be provided in plurality depending on a length of the sterilization module 10.

Referring to FIG. 1, the sterilization module 10 may include the light guide 200. The light guide 200 may be formed with a circular cross section. The light guide 200 may be formed in a cylindrical shape. The light guide 200 may extend in the first direction 12. The light guide 200 may convert the light source module 300, which is a point light source, into a linear light source. The light guide 200 may be formed of a material capable of transmitting light from the light source module 300. The light guide 200 may be formed of a material having light-transmitting properties, which can emit light incident on the interior to the exterior. The light guide 200 may be formed of a material having high transmittance for light generated from the light source 320. The light guide 200 may be formed of a material having high transmittance for ultraviolet wavelengths capable of sterilization. The light guide 200 may be formed of a material having high transmittance for UV-C wavelengths. The light guide 200 may be formed of a material having high transmittance for wavelengths of 100 to 280 nm. The light guide 200 may be formed of quartz. The light guide 200 may evenly diffuse light introduced from the light source module 300 to be described later. The light guide 200 may change a path of light introduced from the light source module 200 to described later.

The light guide 200 may be disposed at the body 100. The light guide 200 may be fixed to the body 100 through the grip member 170 of the body 100.

The light guide 200 may include one end portion 210 disposed at the third side plate 150 of the body 100. One end 210 may include a first side 211 facing the third side plate 150 of the body 100. The light source 320 of the light source module 300 to be described later may be disposed at the first side 211. Through the first side 211, light generated from the light source 320 may be incident on the interior of the light guide 200. The second side 211 may be referred to as a light incident surface.

The light guide 200 may include the other end portion 220 disposed at the fourth side plate 160 of the body 100. The other end portion 220 may be disposed opposite the one end portion 210 in the first direction 12. A fixing member 190 described later may be coupled to the other end portion 220. The other end portion 220 may include a second side 221 facing the fourth side plate 160.

Referring to FIG. 6 or FIG. 7, the light guide 200 may include the first curved section 230 connecting the first side 211 and the second side 221. The first curved section 230 may include a curved surface. The first curved section 230 may have a semicircular shape. The first curved section 230 may be disposed above the second curved section 240. The first curved section 230 may face the open upper side of the body 100. The first curved section 230 may be exposed by the open upper side of the body 100. The first curved section 230 may be referred to as a light emission surface from which light is emitted from the light guide 200.

Referring to FIG. 6 or FIG. 7, the light guide 200 may include the second curved section 240 connecting the first side 211 and the second side 221. The second curved section 240 may be disposed below the first curved section 230. The second curved section 240 may include a curved surface. The second curved section 240 may have a semicircular shape. The second curved section 240 may reflect light incident on the interior of the light guide 200 toward the first curved section 230. The second curved section 240 may change an optical path of light incident in the first direction into the light guide 200. Accordingly, a point light source with strong rectilinear advancing property may be emitted through the first curved section 230. The second curved section 240 may be referred to as a light reflecting surface.

Referring to FIGS. 6, 7, and 10, a reflection pattern 250 may be formed in the second curved line 240. The reflection pattern 250 may include a plurality of reflection patterns 250. The plurality of reflection patterns 250 may be arranged along the first direction 12 of the light guide 200. The plurality of reflection patterns 250 may be spaced apart along the first direction 12. A separation distance d in the first direction 12 between the plurality of reflection patterns 250 may become smaller from the first side 211, where light from the light guide 200 is incident, toward the second side 221. A density of plurality of reflection patterns 250 may increase in a direction away from the light source 320. Accordingly, uniform illuminance may be formed in the entire longitudinal direction of the light guide 200.

The reflection pattern 250 may be formed over an entire area of the second curved section 240. Alternatively, the reflection pattern 250 may be formed in a portion of the second curved section 240.

The reflection pattern 250 may be formed to be embossed or engraved in the second curved section 240. When the reflection pattern 250 is engraved, the reflection pattern 250 may be formed in a groove shape. When the reflection pattern 250 is in an embossed shape, the reflection pattern 250 may be formed in a protrusion shape. Referring to FIGS. 8 and 9, when the reflection pattern 250 is in an embossed shape, the reflection pattern 250 may include a light reflecting member 253. The light reflecting member 253 may protrude upward from an inner peripheral surface of the second curved section 240. The light reflecting member 253 may be formed in various shapes. For example, the light reflecting member 250 may have a cross-section having at least one of a triangular shape, a conical shape, or a hemispherical shape. Hereinafter, an example in which the light reflecting member 250 has a triangular cross-section will be described.

The light reflecting member 253 may include a plurality of light reflecting members 253. The plurality of light reflecting members 253 may be combined to form the reflection pattern 250. The plurality of light reflecting members 253 may be spaced apart along the circumference of the light guide 200 or the longitudinal direction of the light guide 200 in the second curved section 240 to form the reflection pattern 250.

Referring to FIG. 8, the plurality of light reflecting members 253 may be spaced apart along a circumferential direction of the light guide 200 in the second curved section 240 to form a first reflection pattern 251. Referring to FIG. 11, the first reflection pattern 251 may include a plurality of first reflection patterns 251. The plurality of first reflection patterns 251 may be spaced apart along the longitudinal direction of the light guide 200. A separation distance d in the first direction between the plurality of first reflection patterns 251 may increase from the first side 211 of the light guide 200, where light is incident, toward the second side 221 of the light guide 200. Referring to FIG. 11, the light guide 200 may include a first portion 200a, a second portion 200b, and a third portion 200c sequentially arranged from the first side 211 of the light guide 200 along the first direction 12. The first portion 200a of the light guide 200 may be a portion where the separation distance d between the plurality of first reflection patterns 251 is formed as a first separation distance d1. The second portion 200b of the light guide 200 may be a portion where the separation distance d between the plurality of first reflection patterns 251 is formed as a second separation distance d2. The third portion 200c of the light guide 200 may be a portion where the separation distance d between the plurality of first reflection patterns 251 is formed as a third separation distance d2. At this point, the first separation distance d1 may be greater than the second separation distance d2, and the second separation distance d2 may be greater than the third separation distance d3. By arranging the first reflection patterns 251 so that a density between the first reflection patterns 251 increases in a direction away from the light source 320, illuminance of light emitted from the light guide 200 may become uniform. The illuminance of light may become uniform in the longitudinal direction of the light guide 200.

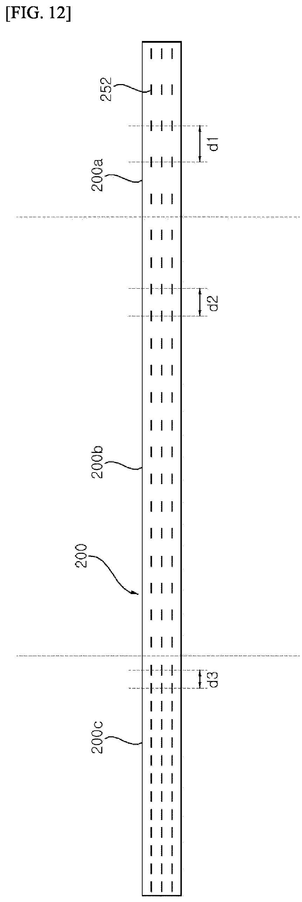

Referring to FIG. 9, the plurality of light reflecting members 253 may be spaced apart along the longitudinal direction of the light guide 200 in the second curved section 240 to form a second reflection pattern 252. Referring to FIG. 12, the second reflection pattern 252 may include a plurality of second reflection patterns 252. The plurality of second reflection patterns 252 may be spaced apart along the longitudinal direction of the light guide 200. The separation distance d between the plurality of second reflection patterns 252 in the first direction may increase from the first side 211 of the light guide 200, where light is incident, toward the second side 221 of the light guide 200. Referring to FIG. 12, the light guide 200 may include a first portion 200a, a second portion 200b, and a third portion 200c sequentially arranged from the first side 211 of the light guide 200 along the first direction 12. The first portion 200a of the light guide 200 may be a portion where the separation distance d between the plurality of second reflection patterns 252 is formed as a first separation distance d1. The second portion 200b of the light guide 200 may be a portion where the separation distance d between the plurality of second reflection patterns 252 is formed as a second separation distance d2. The third portion 200c of the light guide 200 may be a portion where the separation distance d between the plurality of second reflection patterns 252 is formed as a third separation distance d2. At this point, the first separation distance d1 may be greater than the second separation distance d2, and the second separation distance d2 may be greater than the third separation distance d3. That is, by placing the second reflection patterns 252 so that a density between the second reflection patterns 252 increases in a direction away from the light source 320, illuminance of light emitted from the light guide 200 may become uniform.

In addition, referring to FIG. 12, the plurality of second reflection patterns 252 may be spaced apart in a circumferential direction of the light guide 200.

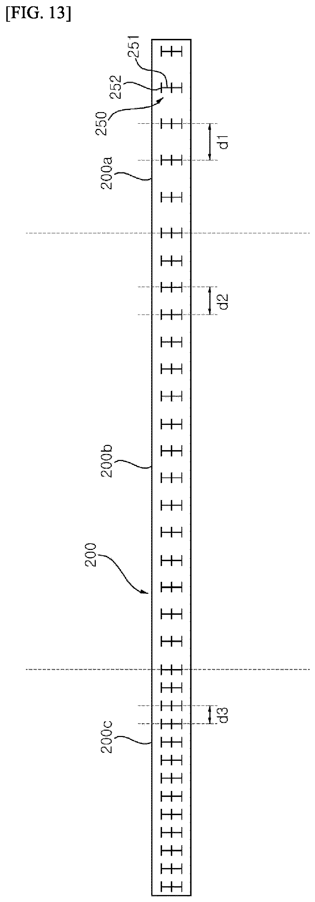

Referring to FIG. 13, a third reflection pattern may be formed by combining the first reflection pattern 251 and the second reflection pattern 252.

Referring to FIGS. 11 to 13, a length of the first portion 200a of the light guide 200 in the first direction 12, a length of the second portion 200b of the light guide 200 in the first direction 12, and a length of the third portion 200c of the light guide 200 in the first direction 12 may be determined based on a desired illuminance. For example, the length of the first portion 200a of the light guide 200 in the first direction 12 may be shorter than the length of the second portion 200b of the light guide 200 in the first direction 12. The length of the first portion 200a of the light guide 200 in the first direction 12 may correspond to the length of the third portion 200c of the light guide 200 in the first direction 12.

Referring to FIG. 10, the reflection pattern 250 may reflect light incident on the light guide 200. The light reflected from the reflection pattern 250 may be directed toward the first curved section 240 of the light guide 200 and may be emitted to the outside of the light guide 200 through the first curved section 240. The light emitted from the light guide 200 may be irradiated to a target material to be sterilized.

Hereinafter, a light loss reduction structure of the light guide 200 is described with reference to the drawings.

Referring to FIGS. 6 and 7, a reflector 180 may be disposed at the body 100. The reflector 180 may be disposed on an inner surface of the body 100. The reflector 180 may be disposed on at least one of the bottom plate 120, the first side plate 130, or the second side plate 140 of the body 100. For example, the reflector 180 may be disposed at the bottom plate 120 of the body 100. For example, the reflector 180 may be disposed at the first side plate 130 and the second side plate 140 of the body 100. For example, the reflector 180 may be disposed at the bottom plate 120 and the first side plate 130. For example, the reflector 180 may be disposed at the bottom plate 120 and the second side plate 1400. For example, the reflector 180 may be disposed at the bottom plate 120, the first side plate 130, and the second side plate 140.

The reflector 180 may surround at least a portion of the light guide 200. The reflector 180 may be disposed to surround the second curved section 240 of the light guide 200. Accordingly, the reflector 180 may reduce light loss in a direction toward the reflection pattern 250. The reflector 180 may reflect transmitted light toward a portion where the reflection pattern 250 is not formed in the second curved section 240. Light reflected by the reflector 180 may be emitted through the first curved section 230 of the light guide 200. The light reflected by the reflector 180 may be irradiated to a target object to be sterilized through the open upper side of the body 100.

The reflector 180 may be formed of a material having a high reflectivity for light generated from the light source 320. The reflector 180 may be formed of a material having a high reflectivity for UV-C wavelengths. The reflector 180 may be formed of a material having a high reflectivity for wavelengths of 100 to 280 nm. The reflector 180 may be formed of aluminum.

In the sterilization module 10 according to embodiments of the present disclosure, the reflector 180 may not be formed in the light guide 200, but formed in the body 100 that fixes the light guide 200, thereby allowing the shape of the reflector 180 to be freely adjusted. A radiation angle of the light guide 200 may be adjusted by adjusting the shape of the reflector 180. Related details thereof will be described later.

FIG. 6 is a view showing an example in which the reflector 180 is applied to the body 100.

Referring to FIG. 6, the reflector 180 may include a first reflector 181 disposed on the first side plate 130. The reflector 180 may include a second reflector 182 disposed on the second side plate 140. The reflector 180 may include a third reflector 183 disposed on the bottom plate 120.

The first reflector 181 may be formed to be inclined inward with respect to an outer surface of the first side plate 130. The first reflector 181 may be formed to be inclined with respect to an axis of the second direction 14. The first reflector 181 may be formed to be inclined downward from the upper side of the body 100 toward the bottom plate 120 of the body 100. A horizontal distance between the first reflector 181 and the outer surface of the first side plate 130 may increase in a direction from the upper side of the body 100 toward the bottom plate 120 of the body 100.

The second reflector 182 may be formed to be inclined inward with respect to an outer surface of the second side plate 140. The second reflector 182 may be formed to be inclined with respect to the axis of the second direction 14. The second reflector 182 may be formed to be inclined downward from the upper side of the body 100 toward the bottom plate 120 of the body 100. A horizontal distance between the second reflector 182 and the outer surface of the second side plate 140 may increase in a direction from the upper side of the body 100 toward the bottom plate 120 of the body 100. A distance between the second reflector 182 and the first reflector 181 may decrease in a direction from the upper side of the body 100 toward the bottom plate 120 of the body 100.

The third reflector 183 may be formed over the entire surface of the bottom plate 120. The third reflector 183 may be connected to the first reflector 181 and the second reflector 182.

The first to third reflectors 181, 182, and 183 may be formed in a trapezoidal shape.

FIG. 7 is a view showing another example in which the reflector 180 is applied to the body 100.

Referring to FIG. 7, at least a portion of the reflector 180 may be formed as a curved surface. A connecting portion between the third reflector 183 and the first reflector 181 may be formed as a curved surface. A connecting portion between the third reflector 183 and the second reflector 182 may be formed as a curved surface. The reflector 180 according to the embodiment of FIG. 7 may be formed in the same manner as the reflector 180 according to the embodiment of FIG. 6, except that a portion is formed as a curved surface.

The reflector 180 may be formed in the body 100 to reflect light, which is not reflected from the second curved section 240 of the light guide 200, toward the second curved section 240 or the open upper side of the body 100, thereby reducing light loss. The reflector 180 may increase light illuminance uniformity. The reflector 180 may adjust a radiation angle of light radiated from the light guide 200. The reflector 180 may adjust a radiation angle of light radiated in the longitudinal direction of the light guide 200. The reflector 180 may adjust a radiation angle of light radiated in the circumferential direction of the light guide 200. In the sterilization module 10 according to embodiments of the present disclosure, since the reflector 180 is formed in the body 100 instead of the light guide 200, the radiation angle and illuminance of light may be controlled by adjusting the shape, position, or the like of the reflector 180.

Meanwhile, in the above description, the reflector 180 is described as being formed in the body 100, but aspects of the present disclosure are not limited thereto, and a reflective coating may be formed by coating the body 100 with a reflective material. In this case, the first side plate 130 of the body 100 may be formed to be inclined downward in a direction from the upper side of the body 100 toward the bottom plate 120 of the body 100, and a reflective coating may be formed on an inner surface of the first side plate 130. The second side plate 140 of the body 100 may be formed to be inclined downward in a direction from the upper side of the body 100 toward the bottom plate 120 of the body 100, and a reflective coating may be formed on an inner surface of the second side plate 140.

Hereinafter, a rotation prevention structure of the light guide 200 will be described with reference to the drawings.

The sterilization module 10 may include the fixing member 190. The fixing member 190 may fix the other end portion 220 of the light guide 200. The fixing member 190 may fix the other end portion 220 of the light guide 200 to prevent rotation of the light guide 200.

Referring to FIGS. 14 and 15, the fixing member 190 and the light guide 200 may be coupled to the body 100. The fixing member 190 may be coupled to the bottom plate 120 of the body 100. The fixing member 190 may be coupled to the fourth side plate 160 of the body 100. A lower surface of the fixing member 190 may be coupled to the bottom plate 120 of the body 100, and one lateral side of the fixing member 190 may be coupled to the fourth side plate 160 of the body 100.

The fixing member 190 may include a groove 191. The groove 191 may be recessed downward from an upper end of the fixing member 190. At least a portion of the other end portion 220 of the light guide 200 may be inserted into the groove 191. The groove 191 may have a shape corresponding to that of the other end portion 220 of the light guide 200.

Referring to FIGS. 14 and 15, a first recessed portion 222 may be formed at the other end portion 220 of the light guide 200. The first recessed portion 222 may be formed at a portion of the other end portion 220 of the light guide 200 that faces the first side plate 130. The first recessed portion 222 may be formed with a flat surface facing the first side plate 130.

A second recessed portion 223 may be formed at the other end portion 220 of the light guide 200. The second recessed portion 223 may be formed at a portion of the other end portion 220 of the light guide 200 that faces the second side plate 140. The second recessed portion 223 may be formed with a flat surface facing the second side plate 240. The second recessed portion 223 may be formed on the opposite side of the first recessed portion 222. The second recessed portion 223 may be symmetrical with the first recessed portion 222.

The other end portion 220 of the light guide 200 may be formed as a curved surface, except for the first recessed portion 222 and the second recessed portion 223.

The groove 191 of the fixing member 190 may be open in a direction toward the open upper side of the body 100. An inner surface of the groove 191 that comes into contact with the first recessed portion 222 may be formed as a flat surface. An inner surface of the groove 191 that comes into contact with the second recessed portion 223 may be formed as a flat surface. The groove 191 may include a bottom surface connecting the inner surfaces in contact with the first and second recessed portions, and the bottom surface of the groove 191 may be formed as a curved surface corresponding to the other end portion 220 of the light guide 200.

The fixing member 190 may include a first fixing part 192, a second fixing part 193 disposed opposite the first fixing part 192, and a connecting part 194 connecting the first fixing part 192 and the second fixing part 193. The first fixing part 192 may be disposed closer to the first side plate 130 than the second fixing part 193. The first fixing part 192 may support the first recessed portion 222. The first fixing part 192 may be in contact with the first recessed portion 222. An inner surface of the first fixing part 192 may be formed as a flat surface corresponding to the first recessed portion 222. The second fixing part 193 may be disposed closer to the second side plate 140 than the first fixing part 192. The second fixing part 193 may support the second recessed portion 223. The second fixing part 193 may be in contact with the second recessed portion 223. An inner surface of the second fixing part 193 may be formed as a flat surface corresponding to the second recessed portion 223. The connecting part 194 may be fixed to the bottom plate 120. The connecting part 194 may be disposed between the first fixing part 192 and the second fixing part 193. A surface of the connecting part 194 that comes into contact with the bottom plate 120 may be formed as a flat surface. A connecting surface of the connecting part 193 connected to the inner surface of the first recessed portion 222 and the inner surface of the second recessed portion 223 may be formed as a curved surface. The connecting surface of the connecting part 193 may be a curved surface having a curvature corresponding to the curvature of the other end portion 220 of the light guide 200.

A shape of the groove 191 may be a 'U' shape. The groove 191 may be a space formed by combining the first fixing part 192, the second fixing part 193, and the connecting part 194.

The fixing member 190 of FIG. 16 and FIG. 17 may be coupled to the light guide 200 and the body 100. The fixing member 190 may be coupled to the bottom plate 120 of the body 100. The fixing member 190 may be coupled to the fourth side plate 160 of the body 100. A lower surface of the fixing member 190 may be coupled to the bottom plate 120 of the body 100, and one lateral side of the fixing member 190 may be coupled to the fourth side plate 160 of the body 100.

The fixing member 190 may include a groove 191. The groove 191 may be recessed downward from an upper end of the fixing member 190. At least a portion of the other end portion 220 of the light guide 200 may be inserted into the groove 191. The groove 191 may have a shape corresponding to that of the other end portion 220 of the light guide 200.

Referring to FIGS. 16 and 17, a recessed portion 224 may be formed at the other end portion 220 of the light guide 200. The recessed portion 224 may be recessed from at least a portion of the other end portion 220 of the light guide 200. The recessed portion 224 may face the bottom plate 120 of the body 100. The recessed portion 224 may be formed as a flat surface. The other end portion 220 of the light guide 200 may be formed as a curved surface except for the recessed portion 224.

The groove 191 of the fixing member 190 may be open in a direction toward the open upper side of the body 100. The groove 191 may be formed with a flat surface in contact with the recessed portion 224. A surface of the groove 191 in contact with the recessed portion 224 may be the bottom surface of the groove 191. The inner surfaces of the groove 191 may be formed as a curved surface having a curvature corresponding to the other end portion 220 of the light guide 200.

The fixing member 190 may include a first fixing part 192, a second fixing part 193 disposed opposite the first fixing part 192, and a connecting part 194 connecting the first fixing part 192 and the second fixing part 193. The first fixing part 192 may be disposed closer to the first side plate 130 than the second fixing part 193. The connecting part 194 may support the recessed portion 224. The connecting part 194 may come into contact with the recessed portion 224. The connecting part 194 may be formed as a flat surface corresponding to the recessed portion 224. The first fixing part 192 may support at least a portion of the side of the other end portion 220 of the light guide 200. The second fixing part 193 may support at least a portion of the side of the other end portion 220 of the light guide 200.

The groove 191 may be a space formed by combining the first fixing part 192, the second fixing part 193, and the connecting part 194.

In the sterilization module 10 according to embodiments of the present disclosure, the light guide 200 formed in a cylindrical shape may be used to convert a point light source into a linear light source, while an rotation prevention structure may be formed at the other end portion 220 of the light guide 200 to prevent rotation of the light guide 200. Specifically, a recessed portion may be formed as a flat surface at the other end portion 220 of the light guide 200, and rotation of the light guide 200 may be prevented through the fixing member 190 having a shape corresponding to that of the other end portion 220 of the light guide 200.

Referring to FIG. 1, the sterilization module 10 may include the light source module 300. The light source module 300 may generate light. The light source module 300 may be disposed at one end portion 210 of the light guide 200 to allow light to be incident on the light guide 200.

The light source module 300 may include the light source 320. The light source 320 may be disposed at the first side 211 of one end portion 210 of the light guide 200. The light source 320 may be disposed between one end portion 210 of the light guide 200 and the third side plate 150 of the body 100. The light source 320 may be disposed at the hole 151 of the third side plate 150 of the body 100. The light source 320 may allow light to be incident on the first side 211 of the light guide 200. The light source 320 may be a UV LED. The light source 320 may generate light having a UV-C wavelength. The light source 320 may generate light having a sterilizing wavelength. The light generated from the light source 320 may have a wavelength of 100 to 280 nm.

The light source 320 may include one light source 320. In the sterilization module 10 according to embodiments of the present disclosure may include one light source 320 and may be configured such that one light source 320 is installed at one end portion 210 of a light guide 200 so that total reflection occurs in the longitudinal direction of the light guide 200. Since the sterilization module 10 according to the embodiment of the present disclosure uses one light source 320, it is possible to reduce a material cost of the sterilization module 10. In addition, it is possible to simplify a wiring structure for generating light from the light source 320.

The light source module 300 may include a substrate 310. The substrate 310 may be a printed circuit board (PCB). The substrate 310 may be a flexible printed circuit board (FPCB). The light source 320 may be mounted on the substrate 310. A connector 330 may be disposed at the substrate 310.

The substrate 310 may include a first portion 311 disposed at the first side plate 130 of the body 100 and a second portion 312 bent from the first portion 311. The first portion 311 may be disposed at the second surface of the first side plate 130. The light source 320 may be mounted on the first portion 311. Solder may be disposed between the light source 320 and the substrate 310. The connector 330 may be disposed at the second portion 312.

The light source module 300 may include the connector 330. The connector 330 may electrically connect an external power source and the substrate 310. The connector 330 may be exposed through a through hole 541 of a cover 300 to be described later. Accordingly, the connector 330 may be electrically connected to the external power source.

Referring to FIGS. 2 to 5, the body 100 may include a rear body 400 in which the light source module 300 is disposed.

The rear body 400 may extend from the body 100 in the first direction 12. The rear body 400 may include a base 410 and first and second extensions 420 and 430 extending upward from both sides of the base 410.

The base 410 may be connected to the third side plate 150. The base 410 may extend from the bottom plate 120. The second portion 132 of the substrate 310 may be disposed at the base 410. The base 410 may have a shape corresponding to that of the second portion 312 of the substrate 310. The base 410 may be covered by a cover 500 to be described later.

The base 410 may include a protruding portion 411 protruding in the first direction 12 from at least a portion of an end of the base 410. The protruding portion 411 may be exposed by the through hole 541 of the cover 500 when the cover 500 to be described is coupled to the rear body 400. The connector 330 may be disposed at the protruding portion 411. At least a portion of the connector 330 may be disposed at the protruding portion 411. The end of the protruding portion 411 may be disposed on the same plane as the end of the connector 330.

The base 410 may be formed with a ring 412 that prevents removal of the substrate 310 disposed at the base 410. The ring 412 may protrude upward from the base 410. The ring 412 may have the protruding portion 411 of the base 410 disposed at both sides. The ring 412 may be positioned further inward than the protruding portion 412. In this case, when the cover 500 is coupled to the rear body 400, the ring 412 may not be exposed due to the cover 500.

The ring 412 may include a first portion 412a protruding upward from the base 410 and a second portion 412b protruding in a direction toward the first side plate 150 from an upper end of the first portion 412a. The first portion 412a may be spaced apart from the end of the second portion 312 of the substrate 310. The second portion 412b may overlap the second portion 312 of the substrate 310 in a vertical direction (second direction 14). Accordingly, the ring 412 may prevent the substrate 310 from being removed.

The first extension 420 may be connected to the first side plate 130. The first extension 420 may be connected to the third side plate 150. The outer surface of the first extension 420 may be positioned further inward than the outer surface of the first side plate 130. An outer surface of the first extension 420 may be positioned further outward than the inner surface of the first side plate 130. The outer surface of the first extension 420 may form a step with the outer surface of the first side plate 130.

The first extension 420 may include a first projection 421 and second projection 422 protruding from an outer surface of the first extension 420. The first projection 421 and the second projection 422 may be spaced apart from each other. An outer surface of the first projection 421 may be disposed on the same plane as an outer surface of the first side plate 130. The outer surface of the second projection 422 may be disposed on the same plane as the outer surface of the first side plate 130. The first projection 421 may be disposed closer to the third side plate 150 than the second projection 422. A first groove 521 of a first side plate 520 of the cover 500 to be described may be disposed at the first projection 421. A second groove 522 of the first side plate 520 of the cover 500 to be described may be disposed at the second projection 422. A portion between the first groove 521 and the second groove 522 of the first side plate 520 of the cover 500 may be disposed between the first projection 421 and the second projection 422.

The first extension 420 may include a third projection 423 protruding from the outer surface of the first extension 420. The third projection 423 may be positioned higher than the first projection 421. The third projection 423 may be positioned higher than the second projection 422. The third projection 423 may be positioned between the first projection 421 and the second projection 422 at a higher position than those of the first and second projections 421 and 422. The third projection 423 may be inserted into a hole 523 of the first side plate 520 of the cover 500 to be described. Accordingly, detachment of the cover 500 may be prevented. The third projection 423 may include an inclined surface. For example, the third projection 423 may have a trapezoidal cross-section. As the third projection 423 has an inclined surface, the position of the cover 500 may be guided when the cover 500 is coupled to the rear body 400.

The first extension 420 may include a first part 424 having a first height in the vertical direction (second direction 14) and a second part 425 having a second height in the vertical direction (second direction 14). The first part 424 may be connected to the first side plate 130. The first part 424 may be connected to the third side plate 150. The first height of the first part 424 may be formed to correspond to a height in a corresponding direction of the third side plate 150. The first height of the first part 424 may have a height corresponding to the height of the first portion 311 of the substrate 310. The first part 424 may overlap with the first portion 311 of the substrate 310. The first part 424 may protrude further than the first portion 311 of the substrate 310 in the horizontal direction (first direction 12). Accordingly, the first portion 311 of the substrate 310 disposed at the third side plate 150 may be protected. The first projection 421 may be disposed at the first part 424.

The second height of the second part 425 may be smaller than the first height of the first part 424. Accordingly, coupling of the cover 500 is easy, and it is convenient for maintenance, such as when there is a short circuit in a circuit part on the substrate 310. The second and third projections 422 and 423 may be disposed at the second part 425.

The second extension 430 may be connected to the second side plate 140. The second extension 430 may be connected to the third side plate 150. The second extension 430 may be disposed opposite the first extension 420. An outer surface of the second extension 430 may be positioned further inward than the outer surface of the second side plate 140. The outer surface of the second extension 430 may be positioned further outward than the inner surface of the second side plate 140. The outer surface of the second extension 430 may form a step with the outer surface of the second side plate 140.

The second extension 430 may have the same configuration as that of the first extension 420. The second extension 430 may have a first projection, a second projection, and a third projection. The second extension 430 may have a first part 434 formed at a first height and a second part 435 formed at a second height lower than the first height.

Referring to FIGS. 2 to 5, the sterilization module 10 may include the cover 500. The cover 500 may be detachably coupled to the rear body 400. The cover 500 may cover the rear body 400. The cover 500 may cover the substrate 310. The cover 500 may cover the first portion 311 and the second portion 312 of the substrate 310. The cover 500 may include an upper plate 510 and first to third side plates 520, 530, and 540 extending downward from the upper plate 510. The upper plate 510 may face the base 410. The cover 500 may have an open lower side. The base 410 may be disposed in the open lower side of the cover 500. The cover 500 has one open lateral side, and the third side plate 150 of the body 100 may be disposed at the open lateral side.

The first side plate 520 may be coupled to the first extension 420. The first side plate 520 may be supported by the first projection 421 and the second projection 422 of the first extension 420. The first side plate 520 may include the first groove 521 and the second groove 522 that are recessed upward from at least a portion of a lower end of the first side plate 520. The first groove 521 may come into contact with the first projection 421 of the first extension 420. The second groove 522 may come into contact with the second projection 422 of the first extension 420. A depth of the first groove 521 may correspond to a height of the first projection 421. A depth of the second groove 522 may correspond to a height of the second projection 422. A portion between the first groove 521 and the second groove 522 of the first side plate 520 may be disposed between the first projection 421 and the second projection 422.

The hole 523 may be formed in the first side plate 520. The third projection 423 of the first extension 420 may be inserted into the hole 523. The hole 523 may be formed at a position corresponding to that of the third projection 423 of the first extension 420. The cover 500 may be prevented from being detached from the rear body 400 by the hole 523 and the third projection 423.

The second side plate 530 may be disposed opposite the first side plate 520. The second side plate 530 may have the same shape as that of the first side plate 520. The second side plate 530 may include a first groove, a second groove, and a hole.

The third side plate 540 may be disposed between the first side plate 520 and the second side plate 530. The third side plate 540 may connect the first side plate 520 and the second side plate 530. The through hole 541 may be formed in the third side plate 540. The through hole 541 may be formed to penetrate the third side plate 540 in the first direction. The through hole 541 may be a groove that is recessed upward from at least a portion of the lower end of the third side plate 540. The connector 330 may pass through the through hole 541. At least a portion of the connector 330 may be exposed through the through hole 541. The end of the connector 330 may be exposed through the through hole 541. Accordingly, the connector 330 may be electrically connected to an external power source.

The sterilization module 10 according to embodiments of the present disclosure may be applied to an air conditioner. The sterilization module 10 according to embodiments of the present disclosure may be applied to a duct-type air conditioner. The sterilization module 10 may be applied to sterilization of a filter in an air conditioner or duct-type air conditioner. However, aspects of the present disclosure are not limited thereto, and the sterilization module 10 according to embodiments of the present disclosure may be applied to sterilization of components requiring sterilization, such as filters and fans. Hereinafter, an example in which the sterilization module 10 is applied to a duct-type air conditioner 1 will be described.

Hereinafter, an air conditioner 1 according to one embodiment of the present disclosure will be described in detail with reference to the drawings.

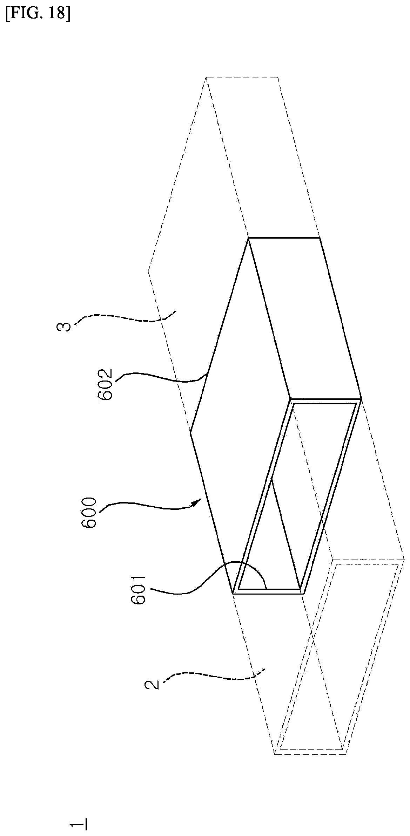

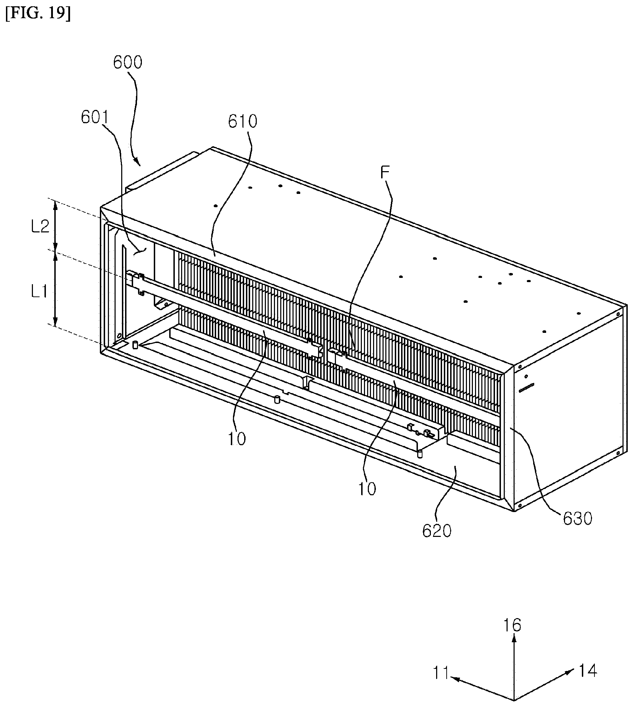

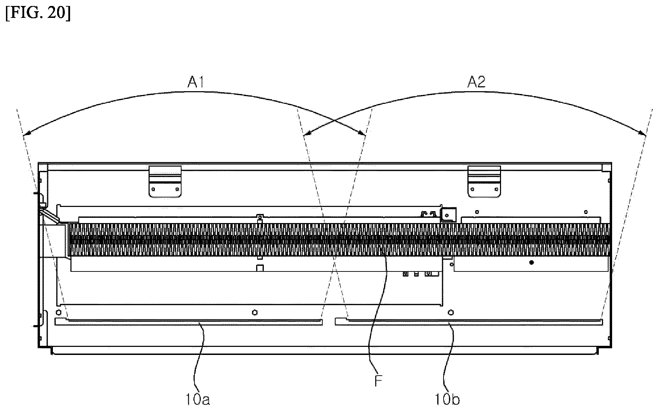

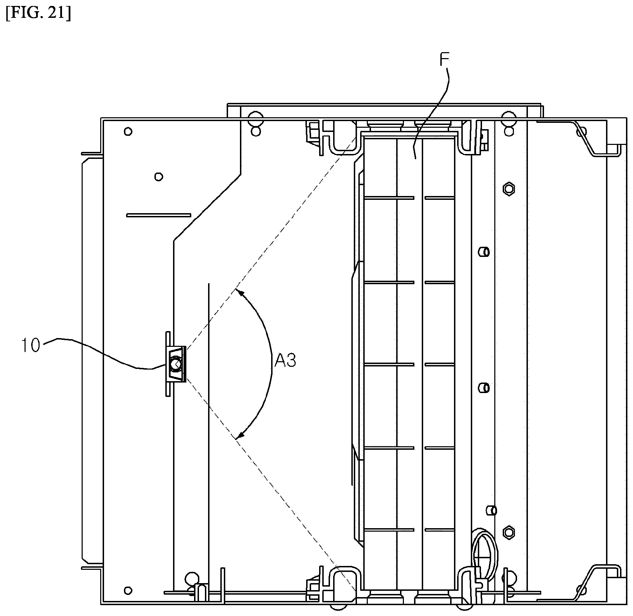

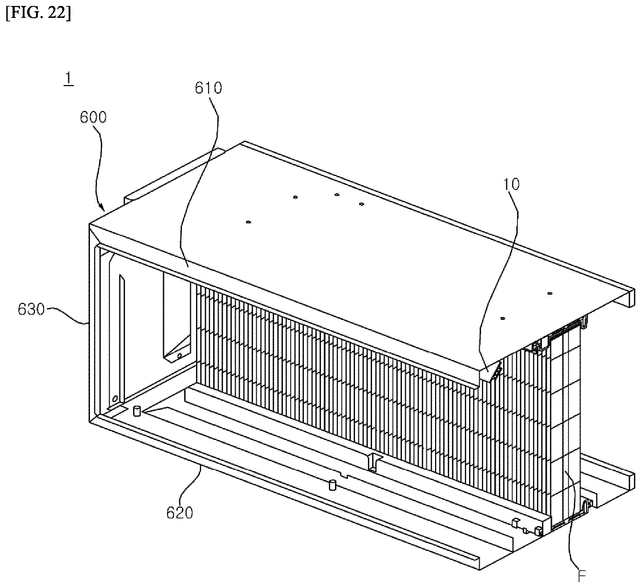

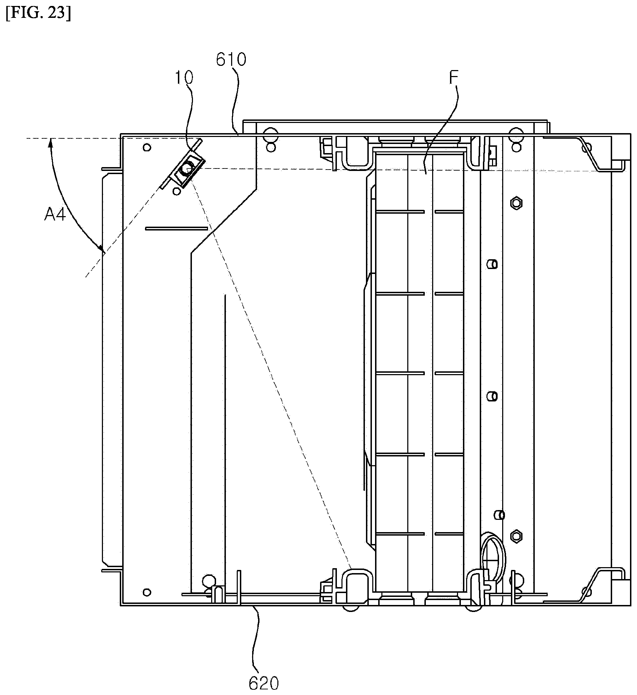

FIG. 18 is a view showing an air conditioner according to one embodiment of the present disclosure. FIG. 19 is a view showing an example of a sterilization module of FIG. 1 being arranged in an air conditioner according to one embodiment of the present disclosure. FIGS. 20 and 21 are views showing a radiation angle of the sterilization module in FIG. 19. FIG. 22 is a view showing another example in which the sterilization module of FIG. 1 is arranged in an air conditioner according to one embodiment of the present disclosure. FIG. 23 is a view showing the radiation angle of the sterilization module in FIG. 22.

Referring to FIG. 18, an air conditioner 1 may be a duct-type air conditioner 1 in which an intake 601 is connected to an suction duct 2 and an outlet 602 is connected to an discharge duct 3. The duct-type air conditioner 1 may have an indoor unit installed in a space within a ceiling or wall. The duct-type air conditioner 1 may be positioned within a wall space without occupying an interior space of a room.

The duct-type air conditioner 1 may include a case 600 including the intake 601 through which air is drawn in, and an outlet 602 formed on the opposite side of the intake 601, through which air is discharged. Within the case 600, a blower fan (not shown) that flows air from the intake 601 to the outlet 6020 may be disposed. Within the case 600. A motor (not shown) that rotates the blower fan (not shown) may be disposed on one side of the blower fan (not shown). A heat exchanger (not shown) that exchanges heat between air flowing through the outlet 602 by the blower fan (not shown) and a refrigerant may be disposed in the case 600. A filter F may be disposed between the blower fan (not shown) and the intake 601. The filter F may be disposed at the intake 601. The duct-type air conditioner 1 may further include a pre-filter (not shown) disposed at front of the filter F to primarily filter out large foreign substances. The filter F, the blower fan (not shown), and the heat exchanger (not shown) may be sequentially arranged in a direction from the intake 601 toward the outlet 602 of the case 600.

The case 100 may include an upper plate 610, a lower plate 620 spaced apart from the upper plate 610 in a third direction 16, and a side plate 630 connecting the upper plate 610 and the lower plate 620. The ase 600 may have a hollow quadrangular prism shape in which in which one side has the inlet 601 and the other side has the outlet 602, both of which are open. One side with the intake 601 and the other side with the outlet 602 may be positioned in opposite directions. Accordingly, an air path extending in a horizontal direction may be formed inside the case 600.

The filter F may be a bent filter. The filter F may be bent into a zigzag shape along a second direction 14 perpendicular to a first direction 12. However, aspects of the present disclosure are not limited thereto, and the filter F may include a filter having a flat surface.

The duct-type air conditioner 1 may include a sterilization module 10. The sterilization module 10 may be disposed between the intake 601 of the case 600 and the filter F. The sterilization module 10 may be disposed in the intake 601 of the case 600. The sterilization module 10 may sterilize the filter F.

The sterilization module 10 may be positioned so that the open upper side of the sterilization module 10 faces the filter F. The sterilization module 10 may be disposed so that a second curved section 240 faces the filter F. The sterilization module 10 may be installed perpendicular to a pleat direction of the filter F. The sterilization module 10 may extend in the first direction 12 perpendicular to the second direction 14. Accordingly, light may be irradiated to the inner side of a bent portion.

Referring to FIG. 19, the sterilization module 10 may be positioned at the center of the intake 601. The sterilization module 10 may be positioned a first distance L1 apart from a lower plate 620 of a case 600 in a vertical direction (third direction 16). The sterilization module 10 may be positioned a second distance L2 apart from an upper plate 610 of the case 600 in the vertical direction (third direction 16). At this point, the first distance L1 and the second distance L2 may be equal to each other. An open upper side of a body 100 of the sterilization module 10 may face the filter F.

The length of the sterilization module 10 in the first direction 12 may correspond to the length of the filter F in the first direction 12. A light guide 200 of the sterilization module 10 may be formed with a length corresponding to a length of the filter F in the first direction 12. In this case, the entire area of the filter F may be sterilized using one sterilization module 10.

A length of the sterilization module 10 in the first direction 12 may be shorter than the length of the filter F in the first direction 12. A length of the light guide 200 in the second direction 12 may be shorter than the length of the filter F in the first direction 12. In this case, a radiation angle of the sterilization module 10 in a longitudinal direction may be adjusted by adjusting the shape, position, or the like of a reflector 180 disposed at the body 100 of the sterilization module 10. Accordingly, the entire area of the filter F longer than the sterilization module 10 may be sterilized.

In addition, if the length of the sterilization module 10 in the first direction 12 is shorter than the length of the filter F in the first direction 12, a plurality of sterilization modules 10 may be used. The plurality of sterilization modules 10 may be spaced apart in the first direction 12. Referring to FIG. 20, for example, the plurality of sterilization modules 10 may include a first sterilization module 10a radiating a first light and a second sterilization module 10b radiating a second light. The first light of the first sterilization module 10a may have a first radiation angle A1 in the longitudinal direction (first direction 12) of the sterilization module 10. The second light of the second sterilization module 10b may have a second radiation angle A2 in the longitudinal direction (first direction 12) of the sterilization module 10. At this point, the first radiation angle A1 and the second radiation angle A2 may overlap at least partially. The first radiation angle A1 and the second radiation angle A2 may be adjusted using the reflector 180. The filter F may include a first region facing the first sterilization module 10a, a second region facing the second sterilization module 10b, and a third region disposed between the first region and the second region and not facing the first and second sterilization modules 10a and 10b. The first light of the first sterilization module 10a and the second light of the second sterilization module 10b may overlap in the third region of the filter F. In the case of the sterilization module 10 according to embodiments of the present disclosure, the reflector 180 is disposed not at the light guide 200, but at the body 100 that fixes the light guide 200, and thus, when the sterilization module 10 is shorter than the length of the filter F in the first direction 12, the light may be radiated beyond the length of the sterilization module 10 by adjusting the reflector 180 or a reflection pattern 250.

The radiation angles A1 and A2 of the sterilization module 10 in the first direction 12 may be adjusted through the reflection pattern 250 of the light guide 200. Referring to FIG. 9, the reflection pattern 250 may include a first surface 253a and a second surface 253b extending in a direction forming a predetermined pattern angle A relative to the first surface 253a. The radiation angles A1 and A2 may increase as the pattern angle A increases. Accordingly, when the sterilization module 10 is shorter than the length of the filter F to be sterilized, the entire area of the filter F may be sterilized by increasing the pattern angle A to increase the radiation angles A1 and A2 in the longitudinal direction.

Conversely, when the sterilization target is shorter than the sterilization module 10, the pattern angle A may be adjusted to narrow the radiation angles A1 and A2, thereby concentrating the light emitted from the sterilization module 10 on the sterilization target to increase the sterilization effect and reduce the light loss.

Referring to FIG. 6 and FIG. 21, the sterilization module 10 may have a radiation angle A3 in a circumferential direction of the light guide 200. Referring to FIG. 6, the radiation angle A3 in the circumferential direction may be an angle between light reflected from one end of the reflection pattern 250 and light reflected from the other end of the reflection pattern 250 in the circumferential direction. The radiation angle A3 in the circumferential direction may be adjusted by the position of the reflection pattern 250 formed in the second curved section 240. For example, a radiation angle A3 (see FIG. 6) formed in a case where the reflection pattern 250 is formed in the entire area of the second curved section 240 may be greater than a radiation angle A3 in a case where the reflection pattern 250 is formed in a portion of the second curved section 240 (an area narrower than the entire area of the second curved section 240). The sterilization module 10 may sterilize the entire area of the filter F in the third direction 16 by adjusting the radiation angle A3 in the circumferential direction.

The sterilization module 10 may be disposed at a position where the first distance L1 is greater than the second distance L2. For example, referring to FIG. 22, the sterilization module 10 may be positioned adjacent to the upper plate 610 of the case 600. The sterilization module 10 may be disposed at an upper end of an inlet 601 of the case 600. In this case, referring to FIG. 23, the sterilization module 10 may be disposed to be inclined at a predetermined angle A4 relative to the upper plate 610 of the case 600. The sterilization module 10 may be disposed to be inclined with respect to the filter F. When the bottom plate 120 of the sterilization module 10 and the upper surface of the sterilization module 10 are formed parallel, the sterilization module 10 and the filter F may form an inclination angle equal to the predetermined angle A4.

While preferred embodiments of the present disclosure have been illustrated and described above, the present disclosure is not limited to the specific embodiments described. Various modifications and implementations can be made by those skilled in the art without departing from the spirit or scope of the present disclosure as claimed in the appended claims. Furthermore, such modifications should not be understood as departing from the technical scope or perspective of the present disclosure.

The present disclosure can be modified and implemented in various forms, and the scope of rights should not be limited to the embodiments described above. Accordingly, any modifications that include the components of the claims of the present disclosure should be considered within the scope of the present disclosure.

The embodiments described above or other examples are not mutually exclusive or distinct. The components or functions of the described embodiments or examples may be combined or used together.

For example, this means that component A described in a particular embodiment and/or drawing may be coupled to component B described in another embodiment and/or drawing. In other words, unless explicitly stated that such coupling of the components are not possible, the coupling should be understood as allowable even if not directly described.

The detailed description provided above should not be interpreted as limiting in any respect but should instead be considered illustrative. The scope of the present disclosure should be determined by the reasonable interpretation of the appended claims, and all modifications within the equivalent scope of the present disclosure should be included.

### [DESCRIPTION OF REFERENCE NUMERALS]

10: sterilization module
100: body
180: reflector
190: fixing member
200: light guide
230: first curved section
240: second curved section
250: reflection pattern
300: light source module

## Claims

1. A sterilization module comprising:
a light source module generating light;
a light guide extending in a first direction and into which light is incident; and
a body having an open upper side and supporting the light guide disposed therein,
wherein the light guide comprises a first side and a second side disposed opposite the first side in the first direction, and
wherein the light source module is disposed at the first side of the light guide to allow light to be incident on the light guide.

2. The sterilization module of claim 1, wherein the light guide has a cross-section formed in a circular shape.

3. The sterilization module of claim 1,
wherein the light source module comprises a light source that generates light having a wavelength of 100 nm to 280 nm, and
wherein the light guide is formed of a material having a high light transmittance.

4. The sterilization module of claim 1,
wherein the light guide comprises:
a first curved section connecting the first side and the second side and formed as a curved surface; and
a second curved section connecting the first side and the second side, formed as a curved surface, and positioned below the first curved section,
wherein a reflection pattern is formed in the second curved section, and
wherein the light incident into the light guide is reflected in the second curved section and emitted to the first curved section.

5. The sterilization module of claim 4,
wherein the reflection pattern comprises a plurality of first reflection patterns extending in the first direction and spaced apart from each other in the first direction, and
wherein a separation distance between the plurality of first reflection patterns decreases in a direction away from the light source module.

6. The sterilization module of claim 4,
wherein the reflection pattern comprises a plurality of second reflection patterns extending in a second direction, which is perpendicular to the first direction and spaced apart from each other in the first direction, and
wherein a separation distance between the plurality of second reflection patterns decreases in a direction away from the light source module.

7. The sterilization module of claim 1, wherein the body comprises a grip member coupled to a bottom plate of the body and gripping the light guide.

8. The sterilization module of claim 1,
wherein the body comprises a bottom plate, a first side plate extending upward from the bottom plate and having the first direction as a longitudinal direction, and a second side plate extending upward from the bottom plate and disposed opposite the first side plate, and
wherein a reflector is disposed at the bottom plate, the first side plate, and the second side plate.

9. The sterilization module of claim 8, wherein the reflector contains aluminum.

10. The sterilization module of claim 8,
wherein the reflector comprises a first reflector disposed at the first side plate and a second reflector disposed at the second side plate, and
wherein a distance between the first reflector and the second reflector in a second direction perpendicular to the first direction increases upward along a third direction perpendicular to both the first and second directions.

11. The sterilization module of claim 8, wherein at least a portion of the reflector is formed as a curved surface.

12. The sterilization module of claim 1,
wherein the light guide comprises one end portion including the first side and the other end portion including the second side,
wherein the sterilization module further comprises a fixing member that fixes the other end portion of the light guide, and
wherein the fixing member is formed with a groove into which the other end portion of the light guide is inserted.

13. The sterilization module of claim 12,
wherein the body comprises a bottom plate, a first side plate extending upward from the bottom plate, a second side plate disposed opposite the first side plate, a third side plate connecting the first side plate and the second side plate and facing the first side of the light guide, and a fourth side plate disposed opposite the third side plate,
wherein the other end portion of the light guide comprises:
a first recessed portion recessed inward from at least a portion of an outer peripheral surface of the other end portion of the light guide and facing the first side plate of the body; and
a second recessed portion recessed inward from at least a portion of the outer peripheral surface of the other end portion of the light guide and facing the second side plate of the body, and
wherein the first recessed portion and the second recessed portion are formed as flat surfaces.

14. The sterilization module of claim 12,
wherein the other end portion of the light guide comprises a recessed portion recessed from at least a portion of an outer peripheral surface of the other end portion of the light guide and faces the bottom plate of the body, and wherein the recessed portion is formed as a flat surface.

15. The sterilization module of claim 1,
wherein the light source module comprises:
a substrate;
a light source mounted on the substrate,
wherein the body comprises a first side plate facing a first side of a light guide,
wherein the first side plate of the body comprises a first surface facing the first side of the light guide, a second surface disposed opposite the first surface, and a hole penetrating the first surface and the second surface,
wherein the substrate is disposed at the second surface of the first side plate of the body, and
wherein the light source is disposed at the hole of the first side plate.

16. The sterilization module of claim 15,
wherein the light source module comprises a connector,
wherein the substrate comprises a first portion disposed at the first side plate of the body, and a second portion bent from the first portion and at which the connector is disposed, and
wherein the sterilization module further comprises a cover coupled to the body and covering the first portion and the second portion of the substrate.
